# EUROPÄISCHE PATENTSCHRIFT

(11) **EP 0 557 670 B1**
(45) Veröffentlichungstag und Bekanntmachung des Hinweises auf die Patenterteilung: **06.05.1999**
(21) Anmeldenummer: 92810133.6
(22) Anmeldetag: 25.02.1992
(51) Int. Cl.: A61K 31/65, A61K 9/50

(54) **Minocyclin enthaltende Pellets**
Pellets containing minocyclin
Pellets contenant de la minocycline

(43) Veröffentlichungstag der Anmeldung: 01.09.1993
(73) Patentinhaber: SPIRIG AG PHARMAZEUTISCHE PRÄPARATE, CH-4622 Egerkingen (CH)
(72) Erfinder: Birrenbach, Gerd, Dr., CH-4616 Kappel (CH); Juch, Rolf-Dieter, CH-4612 Wangen bei Olten (CH)
(74) Vertreter: Braun, André, jr.

(56) Entgegenhaltungen:
- EP-A- 0 327 295
- DE-A- 2 251 197
- LU-A- 84 526

## Beschreibung

Die Erfindung betrifft vom pH unabhängige, den Wirkstoff schnell freigebende, ungecoatete Pellets, die mindestens 90 Gew.-% bis zu 99,5 Gew.-% Minocyclin-HCl oder ein anderes pharmazeutisch verträgliches Minocyclinsalz und gegebenenfalls bis zu 10 Gew.-% Polyethylenglykol (nachfolgend auch PEG genannt) als Pelletierhilfsmittel enthalten. Die Pellets können z.B. in Kapseln abgefüllt werden und werden in der Regel ein- bis zweimal täglich verabreicht.

Minocyclin, insbesondere in Form des Hydrochlorids, wird in grossem Umfang in oralen Dosierungsformen für antibiotische Therapien gegen grampositive und gramnegative Bakterien und Kokken und insbesondere auch zur Aknetherapie eingesetzt, obwohl es Nebenwirkungen aufweist, wie z.B. Magen/Darmbeschwerden, wie Nausea, Dyspepsien und Diarrhoen. Die Beschwerden treten dann bevorzugt auf, wenn das verabreichte Minocyclinpräparat durch längeres Verweilen oder hohe Konzentration an einer bestimmten Stelle im Magen/Darmbereich in der Lage ist, Magen- und Darmschleimhaut zu reizen. Um diese Beschwerden, die vor allem bei der Verabreichung des Arzneimittels auf nüchternen Magen entstehen, zu vermindern, wurde schon versucht, Minocyclin in verschiedenen Vehikeln zu verabreichen. Insbesondere geeignet erschienen dazu bisher auch gecoatete Arzneimittelformen.

Wegen der relativ geringen Stabilität von Minocyclin-HCl und der damit verbundenen begrenzten Verarbeitbarkeit und der kurzen Lagerfähigkeit enthalten Minocyclin-haltige Arzneimittel relativ grosse Mengen an ausgewählt verträglichen Hilfsstoffen, mit deren Hilfe obige Nachteile vermindert werden sollen. Bei den verwendbaren Hilfsstoffen ist jeweils zu beachten, dass sie keine reaktiven Gruppen aufweisen, um unerwünschte Interaktionen zwischen Wirkstoff und Hilfsstoff zu vermeiden. Viele Aezneiformen tragen zudem ein schützendes Coating, sei es zur Stabilitätsverbesserung oder auch, um lokale Reizwirkungen im Magen auf Grund hoher Wirkstoffkonzentrationen zu verhindern.

Studien haben nun entgegen der bisher vertretenen Ansicht ergeben, dass Minocyclin überwiegend im Magen absorbiert wird. Durch eine verzögerte Freigabe des Wirkstoffs wird deshalb ein Teil davon wieder unresorbiert ausgeschieden und belastet unnötigerweise nur den Körper des Patienten, ohne dass die therapeutisch notwendigen Blutspiegelwerte so unter Umständen erreicht werden.

In EP-A-0 418 565 wird ein aufwendiges Minocyclin enthaltendes once-a-day Arzneimittel mit verzögerter Wirkstofffreigabe beschrieben, das ein Hilfsmittel enthaltendes gecoatetes Minocyclingranulat, das den Wirkstoff bei einem pH von 4,0 -7,5 freigibt, und ein Hilfsmittel enthaltendes gegebenenfalls gecoatetes Minocyclingranulat und/oder Minocyclinpulver, das den Wirkstoff bei einem pH von weniger als 3,9 sofort freigibt, enthält. Das Minocyclingemisch kann man einzeln oder zusammen verabreichen. Die Wirkstoffkombination ist somit geeignet, sich im gesamten Magen/Darmtrakt zu lösen. Ein solches Arzneimittel enthält z.B. eine Minocyclin schnell freigebende Komponente, die typischerweise 10-70 Gew.-% Wirkstoff, 90-30 Gew.-% Hilfsstoffe und 0-10 Gew.-% eines Coatings enthält.

In EP-A-0 327 295 wird ein gecoatetes Tetracyclin enthaltendes Arzneimittel beschrieben, das ein Auflösungsprofil bei saurem pH aufweist, welches mindestens demjenigen einer ungecoateten Arzneimittelform entspricht. Unter anderem aus Toxizitätsgründen wird eine 10 %-ige Auflösung bei pH 1,2 nach 1 Stunde und eine 50 %-ige Auflösung bei einem pH 5,5 nach 30 Minuten vorgeschlagen. Durch Benützung von Zuckerkörnern, Bindemitteln, Gleitmitteln und Coatings wird der Wirkstoffanteil, gemessen am Gesamtgewicht des Arzneimittels, auf vorzugsweise 20-75 Gew.-% des Gesamtgewichts des ungecoateten Arzneimittels verringert.

In EP-A-0 310 814 wird ein Granulat mit verzögerter Wirkstofffreigabe beschrieben, das ein Tetracyclin und mindestens ein Hilfsmittel enthält. Das Granulat ist dazu bestimmt, einen kleineren Teil des Wirkstoffs langsam im Magen und den Rest schnell im Darm freizugeben. Ein gegebenenfalls aufgezogenes dünnes Coating aus einem Polymer soll nur langsam im Magen, dafür aber umso schneller im Darmtrakt den Wirkstoff freisetzen. Typischerweise enthält ein solches Arzneimittel etwa 10-70 Gew.-% Wirkstoff und 90-30 Gew.-% Hilfsstoffe.

Aufgabe der Erfindung ist nun die Zurverfügungstellung einer Minocyclinzubereitung, die den Wirkstoff schnell freigibt, pH unabhängig ist, um vergleichbare Werte bei den inter- und intraindividuell unterschiedlichen Resorptionsbedingungen im Patienten beispielsweise vor und nach Nahrungsaufnahme oder auch relativ unabhängig vom Alter zu erhalten, eine im Verhältnis zum Gesamtgewicht der Zubereitung grosse Menge an Wirkstoff enthält und trotzdem lagerstabil ist und geringe Nebenwirkungen aufweist, da sie sich schnell über ein grosses Magenvolumen verteilen kann.

Gelöst wird diese Aufgabe durch das Zurverfügungstellen von vom pH unabhängig Minocyclin freisetzenden, ungecoateten Pellets, die mindestens 90 Gew.-% bis zu 99,5 Gew.-% Minocyclin-HCl oder ein anderes pharmazeutisch verträgliches Minocyclinsalz und 0,5 Gew.-% bis zu 10 Gewichts-% Polyethylenglykol als Pelletierhilfsmittel und gegebenenfalls Politur enthalten.

Die Pellets werden zweckmässigerweise in einem Hochgeschwindigkeitsmischer durch Aufsprühen eines Polyethylenglykol enthaltenden Wasser oder Wasser/Alkoholgemisch auf das feinkörnige Minocyclin-HCl oder ein anderes pharmazeutisch verträgliches Minocyclinsalz hergestellt. Die Partikelgrösse des verwendeten Minocyclins-HCl ist vorteilhafterweise unter 200 µm, doch kann diese wegen der sofortigen Auflösung des Minocyclins-HCl im Magen in weiten Bereichen ohne Veränderung der pharmakokinetischen Eigenschaften variieren.

Als Hochgeschwindigkeitsmischer eignen sich bevorzugt z.B. ein Rotorprozessor, wie der Aeromatic-Rotorprozessor (von Niro-Aeromatic, Bubendorf, Schweiz), oder ein Flügelmischer, wie z.B. der Diosnamischer (von Dirks, Osnabrück, Deutschland), oder ein Lödigemischer (von Lödige, Paderborn, Deutschland). Das Herstellungsverfahren wird durch wenige Parameter, wie z.B. die Temperatur, Prozessluftmenge (dies gilt nicht für die Herstellung mit einem Flügelmischer), relative Feuchte, Sprührate und Rotationsgeschwindigkeit des Mischers gesteuert. Damit werden von Ansatz zu Ansatz reproduzierbare Pellets und üblicherweise Ausbeuten von über 70 % in den gewünschten Pelletgrössen von 0,5-1,4 mm vorzugsweise 0,8-1,2 mm erhalten. Übergrössen können vermahlen und mit den Untergrössen in weiteren Ansätzen verarbeitet werden. Das Verfahren an sich wird kontrolliert durch die Temperaturen der Zu- und Abluft und des Produkts, den Druck, mit welchem das Lösungsmittel und gegebenenfalls das Pelletierhilfsmittel auf den Wirkstoff aufgesprüht wird, und dessen Sprührate (d.h. dessen aufgesprühte Menge), die Drehzahl des Mischers und gegebenenfalls die Menge der Prozessluft.

Das für galenische Zwecke besonders geeignete Wasser, Alkohol oder ein Gemisch von beiden eignen sich gut als Lösungsmittel. Als Alkohole werden vorzugsweise die in der Galenik gebräuchlichen niederen Alkohole, wie Ethanol oder Isopropanol, ein Gemisch davon oder ein Gemisch davon mit Wasser verwendet. Besonders gut eignet sich die Verwendung von Wasser.

Polyethylenglykol als Pelletierhilfsmittel erleichtert die Herstellung wohlgerundeter Pellets, fördert die Löslichkeit bei verschiedenen pH-Werten gleichermassen und begünstigt dadurch die Verfügbarkeit von Minocyclin-HCl im Magen. Überraschenderweise hat es sich gezeigt, dass Polyethylenglykol als relativ reaktiver mehrwertiger Alkohol bei der erfindungsgemässen Anwendung und ohne zusätzliche Verwendung von z.B. Klebern, Bindemitteln oder Stabilisatoren durchaus in der Lage ist, die Stabilität von Minocyclin-HCl nicht negativ zu beeinflussen. Zudem reicht es aus, wenn nicht mehr als 10 Gewichts-% an Polyethylenglykol, vorzugsweise nicht mehr als 7 Gewichts-%, besonders bevorzugt weniger als 4 Gew.-%, verwendet wird, um die minutenschnelle und vollständige Auflösung der Pellets im Magensaft zu gewährleisten und trotzdem gegen Umwelteinflüsse beständige Pellets zu erhalten. Auf diese Art erhält man somit unabhängig vom pH auflösbare und alterungsbeständige Pellets, die übliche und sinnvolle Haltbarkeitszeiten für Arzneimittel erreichen.

Es können verschiedene Polyethylenglykole, wie z.B. Polyethylenglykol 2000, 3000, 4000, 6000, 10000 oder 20000 oder Gemische davon verwendet werden. Vorzugsweise wird Polyethylenglykol 4000 oder eine Kombination aus Polyethylenglykol 4000 und Polyethylenglykol 10000 verwendet.

Besonders bevorzugt sind Pellets, die 6,8 Gew.-% Polyethylenglykol 4000 oder 3,9 Gew.-% Polyethylenglykol 4000 und 2,9 Gew.-% Polyethylenglykol 10'000 enthalten.

Um eine bessere Endrundung und Friabilität der Pellets zu erhalten und den Abfüllvorgang der Pellets in die Kapseln zu erleichtern, können die Pellets in einem weiteren Verfahrensschritt mit geringen Mengen Polyethylenglykol leicht poliert werden.

Die neuen Pellets können zwar auch direkt eingenommen werden, doch ist es vorteilhaft, diese in vordosierter Form zu verabreichen. Zweckmässigerweise werden die Pellets in Kapseln abgefüllt, vorzugsweise in Gelatinekapseln. Sie können aber auch mit geeigneten Hilfsstoffen ohne wesentliche Beschädigung zu Tabletten verpresst werden. Eine Zubereitung kann bis zu 200 mg Minocyclinpellets, vorzugsweise etwa 50-100 mg, enthalten.

Vorteilhaft sind Pellets, die mindestens 93 Gew.-% Minocyclin-Hydrochlorid und höchstens 7 Gew.-% Polyethylenglykol enthalten.

Besonders vorteilhaft sind Pellets, die mindestens 96 Gewichts-% Minocyclin-HCl und nicht mehr als 4 Gewichts-% Polyethylenglykol enthalten. Diese zu etwa je 52 oder 104 mg in Hartgelatinekapseln beispielsweise der Grösse 5 resp. der Grösse 4 abgefüllt, ergeben die bevorzugte Zubereitung. Sie kann - auf Grund der Kleinheit der Kapseln - bei bester Akzeptanz ein- oder zweimal täglich dem Patienten verabreicht werden, wobei die Plasmakonzentration des Minocyclins den therapeutischen Bereich sicher erreicht, aber auch toxische Konzentrationen vermieden werden.

Ein übliches Therapieschema kann am ersten Tag eine zweimalige Verabreichung von je 100 mg Minocyclin-HCl, gefolgt von zweimal je 50 mg in den folgenden Tagen oder jeweils 100 mg morgens und abends 50 mg enthalten.

Es hat sich zudem gezeigt, dass durch Verwendung der erfindungsgemässen Pellets die inter- und intraindividuellen Plasmaspiegelschwankungen von Probanden gegenüber dem Stand der Technik überraschenderweise signifikant verringert werden.

Die erfindungsgemässen Pellets zeichnen sich aus durch eine praktisch vollständige und pH-unabhängige in vitro-Freigabe des Wirkstoffs innert weniger als 10 Minuten, vorzugsweise in weniger als 5 Minuten, sowohl bei pH 1,2 als auch bei pH 7,4.

Die Erfindung wird in den folgenden Beispielen näher erläutert.

### Beispiel 1

Ausgangsstoffe für eine Charge von 100 kg Pellets:

| | |
|---|---|
| a) Minocyclin-HCl | 97,0 kg |
| b) PEG 2000 | 3,0 kg |
| c) Aqua pur. | q.s. |

b) wird in c) gelöst. Dann wird a) in einem Hochgeschwindigkeitsmischer (Rotorprozessor von Aeromatic) vorgelegt und mit der Lösung aus b)+c) bei einer Temperatur von unter 40°C während 30 Minuten besprüht. Der Ansatz wird darauf solange mit Wasser besprüht, bis Pellets in der Grösse von 0,8-1,2 mm entstanden sind. Dies dauert etwa 60 Minuten. Die Pellets werden sodann bei 50°C im Mischer getrocknet und die gewünschte Pelletgrösse durch Sieben abgetrennt.

Eine Ausbeute von mehr als 70 kg Pellets der gewünschten Grösse wird erhalten, die einen Gewichtsanteil von 97,0 % an Minocyclin-HCl aufweisen. Die Über- und Untergrössen der Pellets können in weiteren Chargen verwendet werden.

Die Pellets weisen eine geringe Friabilität und eine Bulkdichte (auf 100 g) von 135-155 ml auf. Dies erleichtert die maschinelle Abfüllung in kleinste Hartgelatinekapseln.

Die Pellets werden so auf einer automatischen Abfüllmaschine in Hartgelatinekapseln der Grössen 5 oder 4 abgefüllt. Eine Kapsel enthält 50 oder 100 mg Minocyclin-HCl und 1,5 oder 3 mg Polyethylenglykol.

### Beispiel 2

Das Verfahren von Beispiel 1 wird wiederholt mit der Ausnahme, dass 99,5 kg Minocyclin-HCl mit 0,5 kg PEG 3000 in einem Diosnamischer besprüht werden.

Es werden Pellets mit einem Minocyclin-HCl Gewichtsanteil von 99,5 % erhalten.

### Beispiel 3

Das Verfahren von Beispiel 2 wird wiederholt mit der Ausnahme, dass 96 kg Minocyclin-HCl und 4 kg PEG 4000 als Pelletierhilfsmittel verwendet werden.

Es werden Pellets mit 96 Gew.-% Minocyclin-HCl erhalten.

### Beispiel 4

Das Verfahren von Beispiel 1 wird wiederholt mit der Ausnahme, dass 90 kg Minocyclin-HCl und 4 kg PEG 6000 als Pelletierhilfsmittel verwendet werden.

Darauf werden 6 kg PEG 20000 in Aqua pur. gelöst und unter ständigem Mischen während 15 Minuten auf die Pellets aufgesprüht, die dann nochmals bei 50°C getrocknet werden.

Es werden Pellets mit 90 Gew.-% Minocyclin-HCl erhalten.

### Beispiel 5

Das Verfahren von Beispiel 2 wird wiederholt mit der Ausnahme, dass 94 kg Minocyclin-HCl und 3 kg PEG 3000 als Pelletierhilfsmittel verwendet werden.

Darauf werden 3 kg PEG 10000 in Aqua pur. gelöst und unter ständigem Mischen während 15 Minuten auf die Pellets aufgesprüht, die dann nochmals bei 50°C getrocknet werden.

Es werden Pellets mit 94 Gewichts-% Minocyclin-HCl erhalten.

### Beispiel 6

Das Verfahren von Beispiel 4 wird wiederholt mit der Ausnahme, dass 93,2 kg Minocyclin-HCl und 3,9 kg PEG 4000 als Pelletierhilfsmittel verwendet werden und in einem zweiten Schritt 2,9 kg PEG 10000 aufgesprüht werden.

### Beispiel 7

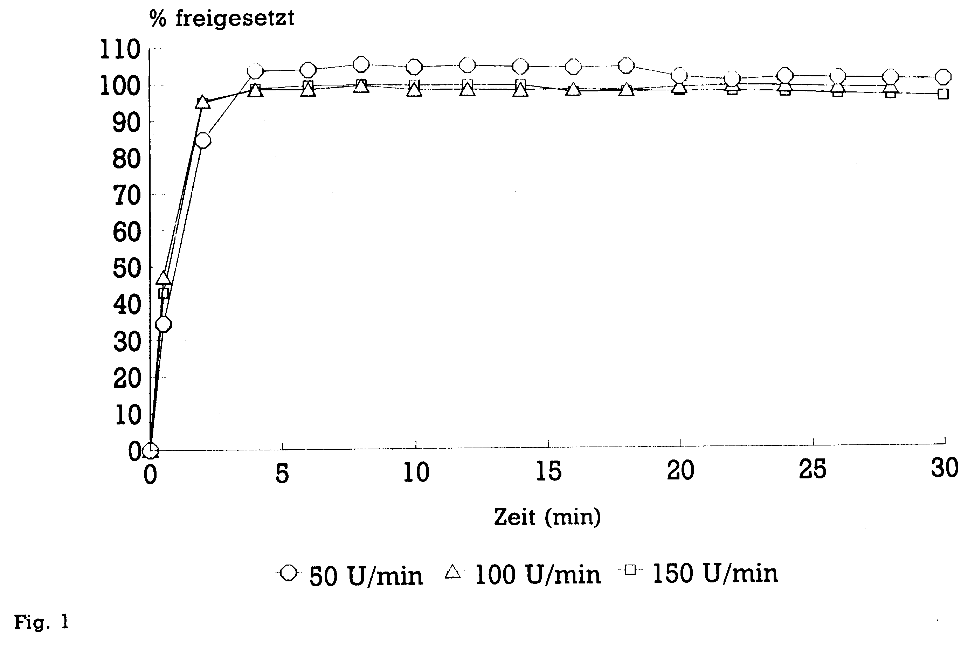

Das Freigabeverhalten verschiedener gemäss Beispiel 3, 4 und 6 hergestellter Chargen wird gemäss USP XXIII unter Benützung von 900 ml gepufferter wässriger Lösung durchgeführt. Die Resultate sind in den Fig. 1-6 aufgezeichnet.

In Fig. 1 wird das Freigabeverhalten von Minocyclin-HCl aus Pellets, hergestellt gemäss Beispiel 3, bei pH 1,2 bei 50 U/Min, 100 U/Min und 150 U/Min der Paddle aufgezeichnet. Es kann gezeigt werden, dass die Freigabe nicht von der Drehzahl der Paddle abhängt.

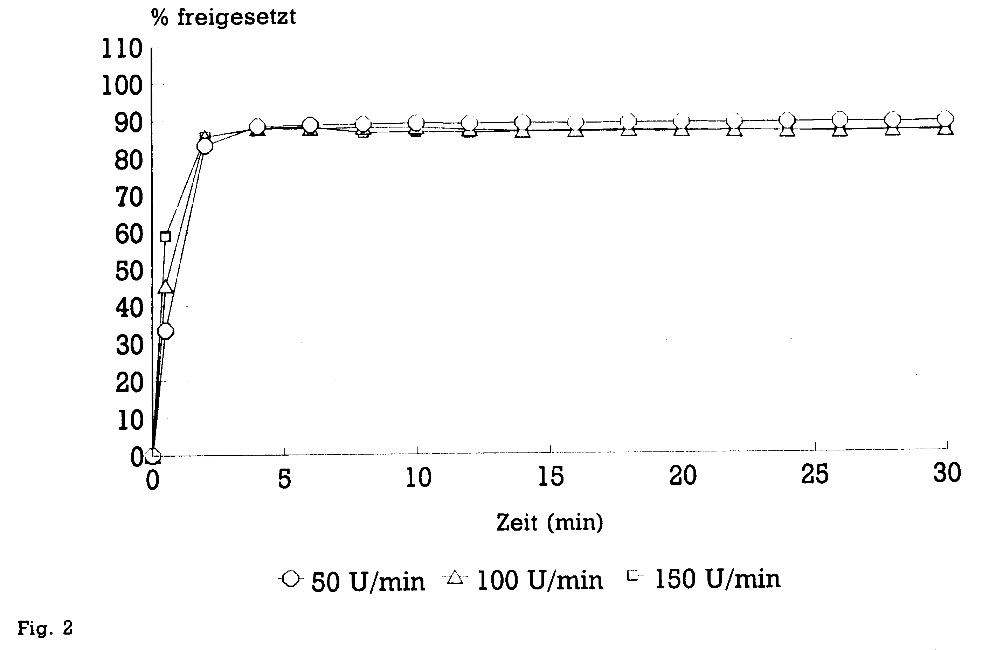

In Fig. 2 wird das Freigabeverhalten analog zu Fig. 1 untersucht, mit der Ausnahme, dass dies bei pH 7,4 durchgeführt wird. Die pH-unabhängige Freisetzung in vitro ist somit offensichtlich. Auch bei diesem pH ist die Freigabe von Minocyclin nicht von der Drehzahl der Paddle abhängig. Man kann somit erwarten, dass die Freisetzung in-vivo im Magen/Darmtrakt bei unterschiedlichem pH gleichermassen verläuft und auch von der Motilität unbeeinflusst bleibt.

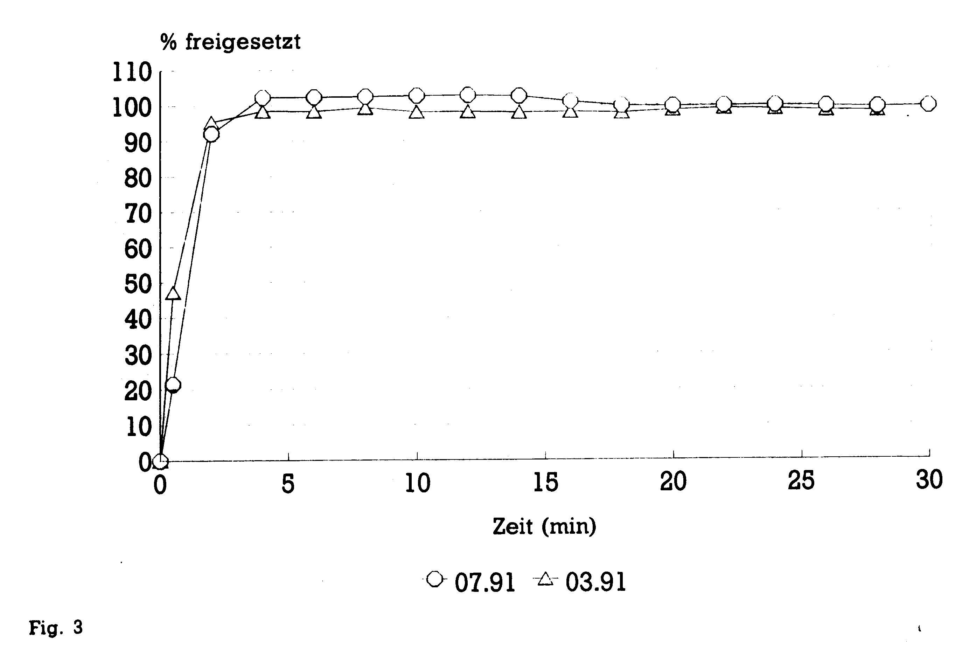

In Fig. 3 wird das Freigabeverhalten von Minocyclin-HCl aus Pellets, hergestellt gemäss Beispiel 3, bei pH 1,2 nach der Herstellung und nach viermonatiger Lagerung gezeigt. Das Freigabeverhalten verändert sich nicht signifikant.

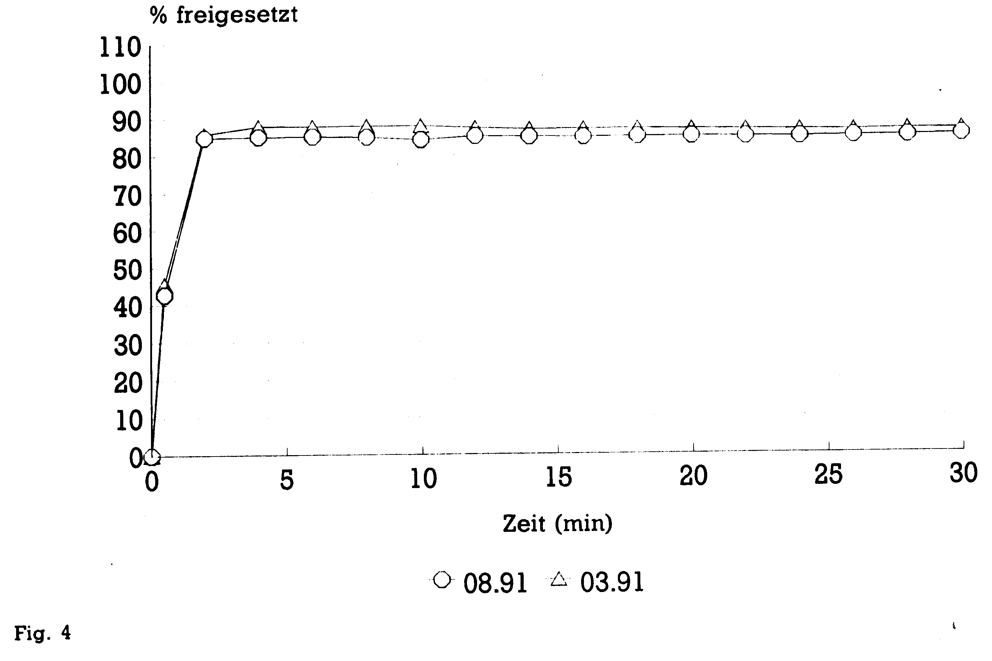

In Fig. 4 wird das Freigabeverhalten analog zu Fig. 3 untersucht, mit der Ausnahme, dass dies bei pH 7,4 und nach fünfmonatiger Lagerung durchgeführt wird. Das Freigabeverhalten verändert sich nicht signifikant, was dem Nachweis der Stabilität und Lagerungsfähigkeit der erfindungsgemässen Pellets dient.

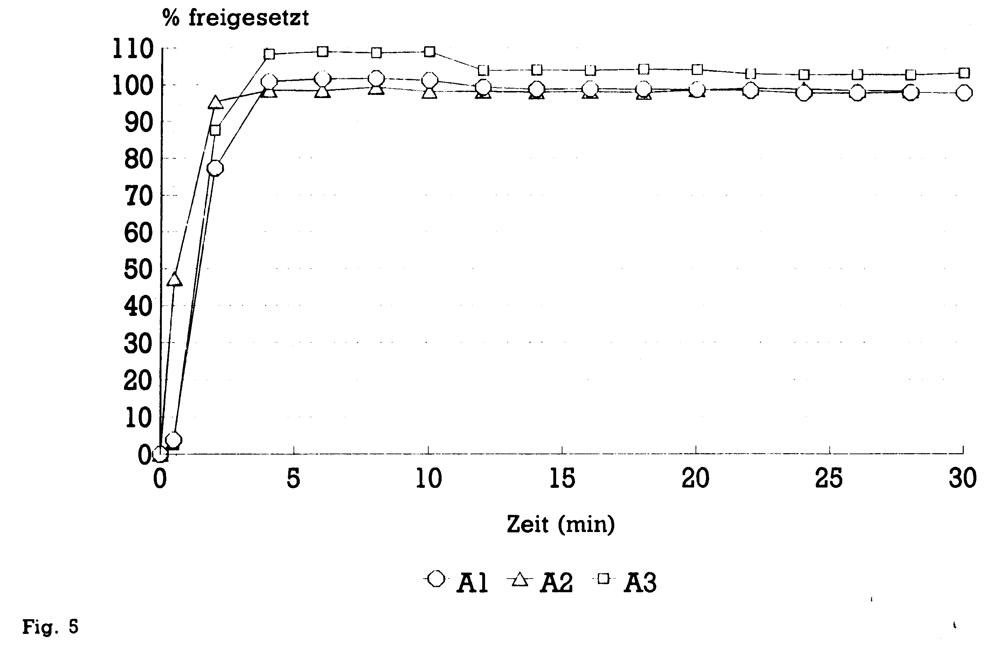

In Fig. 5 wird das Freigabeverhalten dreier verschiedener Ansätze, nämlich A1 (hergestellt nach Beispiel 4), A2 (hergestellt nach Beispiel 3) und A3 (hergestellt nach Beispiel 6) bei pH 1,2 und 100 U/Min gezeigt. Alle drei Ansätze haben vergleichbare und nicht signifikant verschiedene Freigabeverhalten, was beweist, dass die erfindungsgemässen Pellets reproduzierbare Freigabewerte aufweisen.

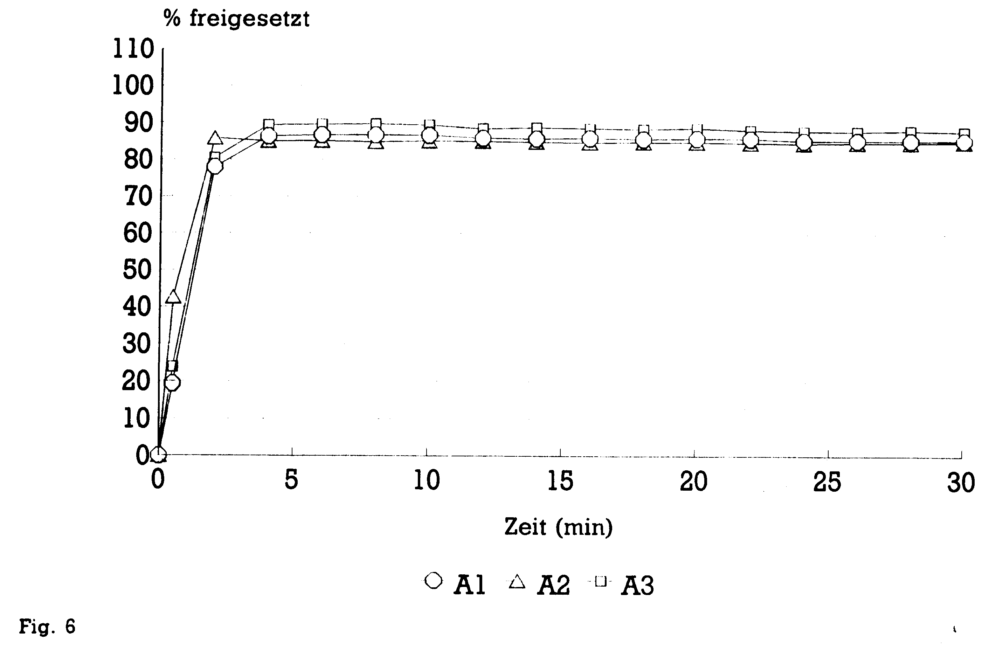

In Fig. 6 wird das Freigabeverhalten analog zu Fig. 5 untersucht, mit der Ausnahme, dass dies bei pH 7,4 durchgeführt wird. Alle drei Ansätze haben vergleichbare und nicht signifikant verschiedene Freigabeverhalten, was beweist, dass die Pellets reproduzierbare Freigabewerte unabhängig vom pH aufweisen.

Aus den Darstellungen in Fig. 1-6 wird ohne weiteres ersichtlich, dass das Freigabeverhalten der erfindungsgemässen Pellets alterungsstabil und pH-unabhängig ist, d.h. dass die Pellets beste Voraussetzungen aufweisen, als Arzneimittel gewisse Zeit gelagert werden zu können, und sowohl auf nüchternen Magen als auch nach dem Essen zu sich genommen werden können, und dass die Pellets Minocyclin-HCl in jedem Fall und unabhängig vom vorherrschenden pH und der Magen-Darmmotilität innert weniger Minuten praktisch vollständig freisetzen.

### Beispiel 8

Es wurde eine Minocyclinzubereitung gemäss Beispiel 3 hergestellt, die pro Dosierungseinheit 100 mg Minocyclin-HCl enthält. Die Pellets wurden in Hartgelatinekapseln als Dosierungseinheit abgefüllt.

Eine Studie wurde zum Zwecke der Bestimmung der Bioverfügbarkeit und Untersuchung der bekanntermassen problematischen Schwankungen der Plasmawerte der Probanden durchgeführt. Der Vergleich wurde an 6 gesunden männlichen Probanden im Alter von 19-35 Jahren (No. 1 bis No. 6) angelegt.

Die Plasmawerte wurden mittels HPLC bestimmt. Verwendet wurde eine Nucleosil 10 C₈ Säule und eluiert wurde mit Dimethylformamid, Ammoniumoxalat/Natriumedetat 0,1M.

Den am Testtag in der Frühe nach achtstündiger Nüchternphase erschienenen Probanden wurde nach Abnahme des Leerblutes je eine Kapsel verabreicht. Die weiteren Blutentnahmen erfolgten 0,5, 1, 2, 3, 4, 6, 8, 12, 24 und 36 Stunden nach Einnahme der Kapsel.

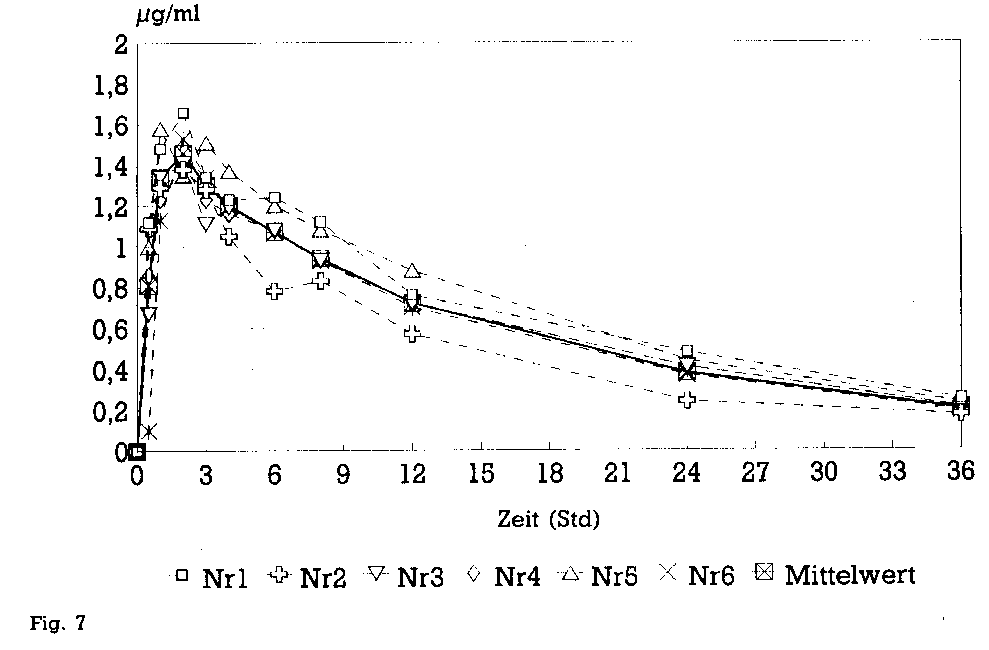

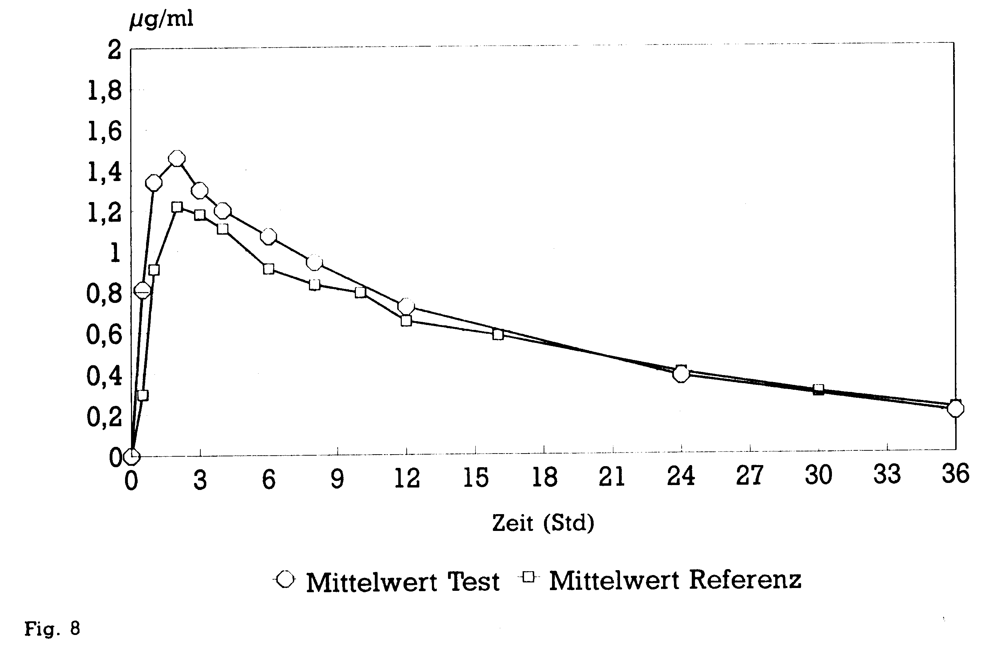

Die Auswertung des Versuchs ergab, dass mit der erfindungsgemässen Zubereitung überraschende Vorteile erreicht wurden:
- Die Plasmaspiegel der Probanden zeigen extrem geringe Streuungen um den Durchschnittswert herum auf und weisen auf eine gute Reproduzierbarkeit der Pharmakokinetik hin (Fig. 7).
   Der Variationskoeffizient (Standardabweichung x 100/Mittelwert) als Mass für die Streuung liegt mit 13,4 % aussergewöhnlich niedrig für diese Stoffklasse. Betrachtet man beispielsweise die Streuung beim stoffgruppenverwandten Doxycyclin mit bekanntermassen grossen Schwankungen der individuellen Plasmawerte, so hat man hier die für eine Beurteilung der relativen Bioverfügbarkeit üblichen Grenzen von 80-120 % auf einen breiteren Bereich von 70-130 % festgelegt: man akzeptiert also Abweichungen von +/- 30 % der wahren relativen Bioverfügbarkeit des Testpräparates vom Referenzprodukt unter vergleichbaren Bedingungen (H. Blume et al., Deutsche Apotheker Zeitung, 127, 1987, 2090 -2094).
- Ein Vergleich der erfindungsgemässen Pellets mit einem der führenden Handelspräparate in Pelletform (Fig. 8) zeigt, dass mit erfindungsgemässen Pellets höhere Blutspiegelwerte erhalten werden, diese sich nach ca. 15 Stunden wieder einander angleichen und beide über ca. 24 Stunden Plasmawerte im therapeutischen Bereich von etwa 0,1-1,0 µg/ml erreichen. Es zeigt sich weiter, dass die erfindungsgemässen Pellets schneller verfügbar sind und, durch Messung der Fläche unter den beiden Kurven, dass die Gesamtmenge an resorbiertem Wirkstoff dabei höher als bei dem Handelsprodukt ist.

### Vergleichsbeispiel

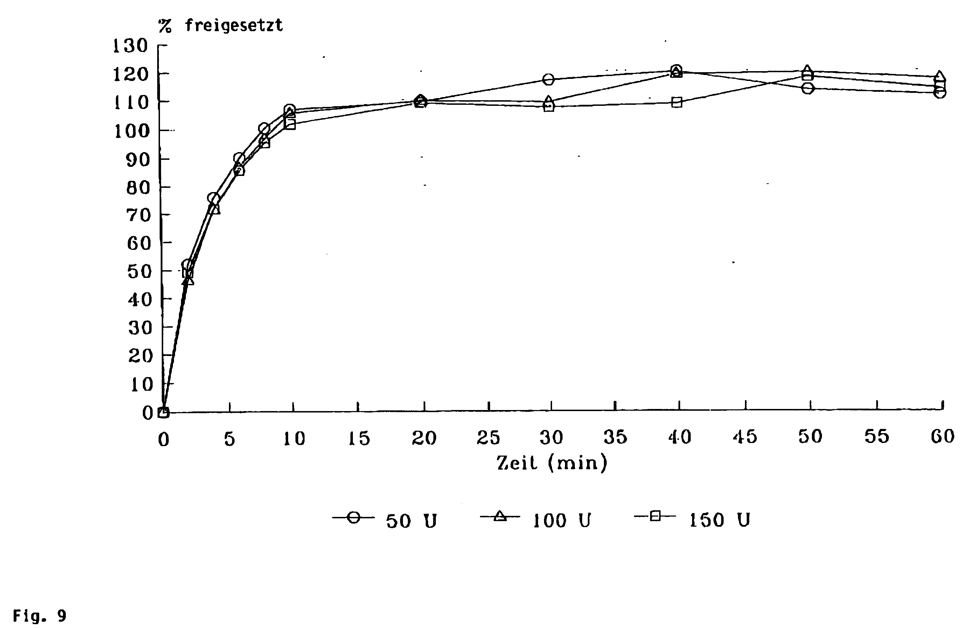

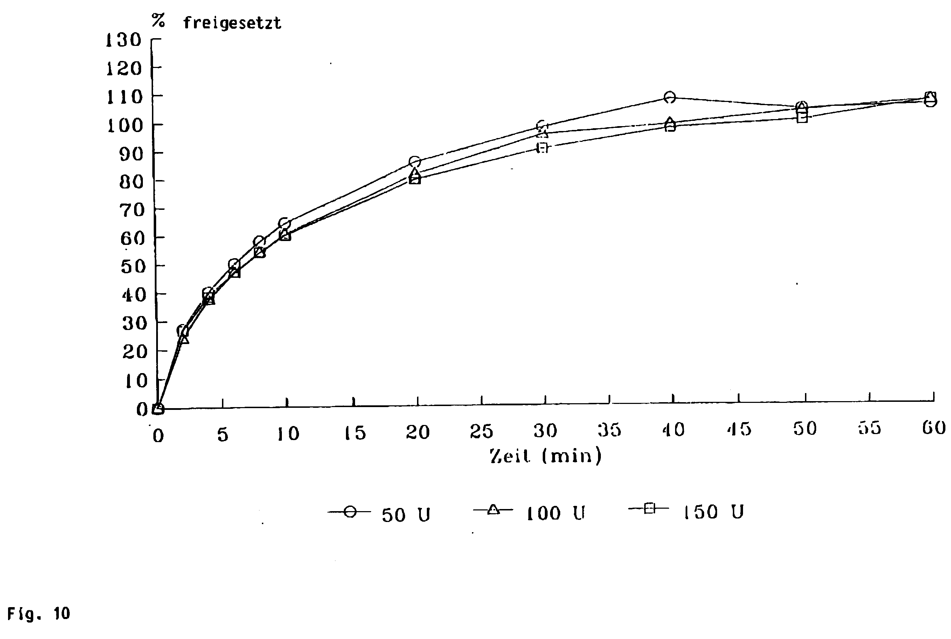

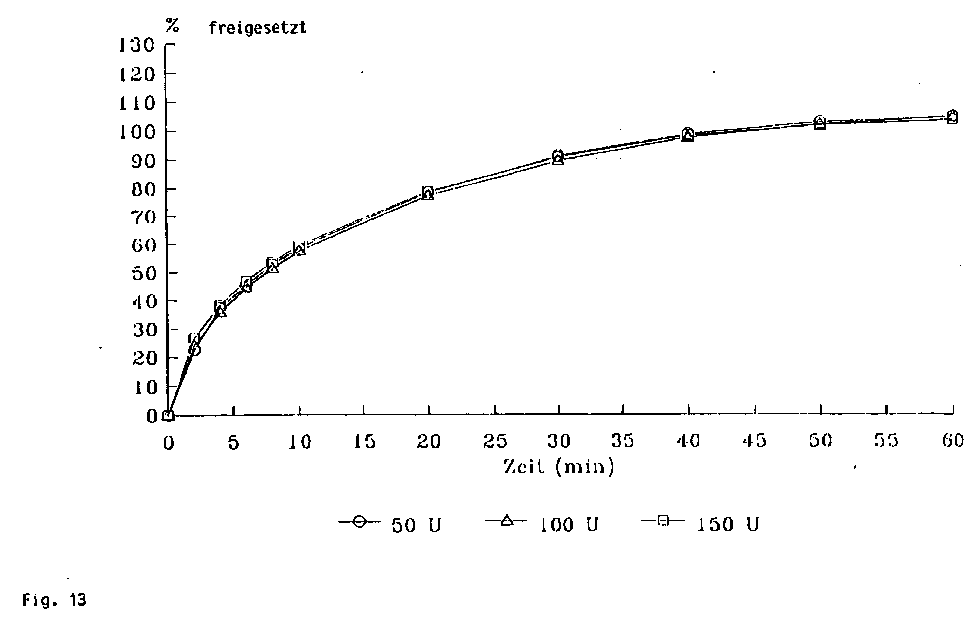

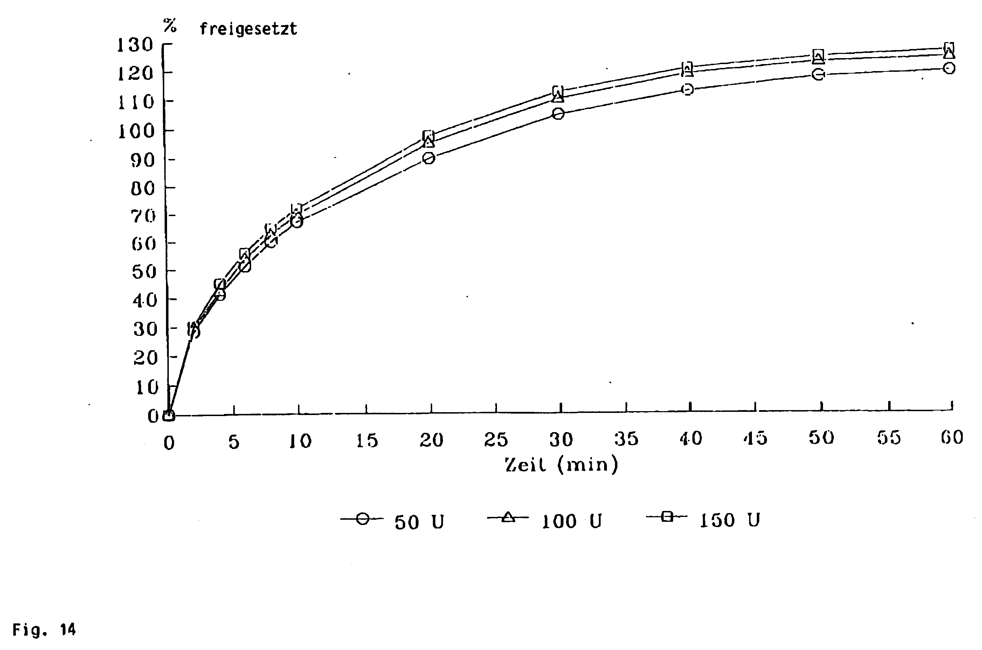

Die in vitro-Freigabe von erfindungsgemässen Minocyclin-HCl-Pellets, wie sie im Beispiel 7 beschrieben ist, wurde mit der in vitro-Freigabe von Minocyclin-HCl-Pellets, die als Handelsprodukt der Firma Lederle, USA, unter der Marke Minocin verkauft werden, verglichen. Unter gleichen Versuchsbedingungen wie in Beispiel 7 wurden Minocinkapseln mit 50 mg und mit 100 mg Minocyclin-HCl untersucht. Zusätzlich wurden Freigabeversuche in gepufferter Lösung durchgeführt. Die Resultate sind in den Fig. 9-14 festgehalten.

In Fig. 9 wird das Freigabeverhalten von 100 mg Minocyclin-HCl-Pellets bei pH 1,2 bei 50 U/Min, 100 U/Min und 150 U/Min der Paddle aufgezeichnet.

In Fig. 10 wird das Freigabeverhalten analog zu Fig. 9 untersucht, mit der Ausnahme, dass dies bei pH 7,4 durchgeführt wird. Dabei zeigt es sich, dass die Freisetzung offensichtlich pH-abhängig ist, da bei diesem pH die Freigabe so stark verzögert wird, dass erst nach ca. 45 Minuten mit einer 100% Freigabe von Minocyclin-HCl gerechnet werden kann.

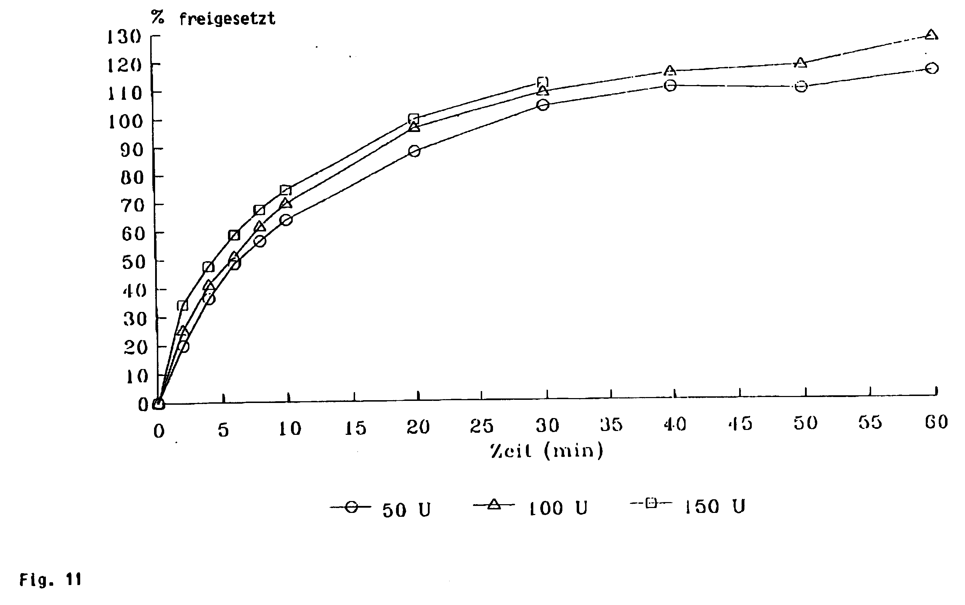

Diese Angaben werden durch die in Fig. 11 vorgenommenen Messungen erhärtet. Auch in einer mit pH 5,6 noch schwach sauren Pufferlösung wird die Freigabe des Minocyclin-HCl verzögert. Mit einer 100 % Freigabe kann erst nach mindestens 30 Minuten gerechnet werden. Da die Magenverweilzeit von Pellets in der Regel aber kleiner als 30 Minuten ist, kann es bei ungünstiger Ausgangslage, d.h. relativ hohem pH im Magen, vorkommen, dass ein wesentlicher Teil des Wirkstoffs am Resorptionsorgan Magen vorbeigeschleust wird.

Man kann somit erwarten, dass die Freigabe des Minocyclin-HCl aus Minocinkapsel auch in-vivo im Magen/Darmtrakt bei unterschiedlichem pH unterschiedlich verläuft, so dass eine vollständige Resorption des Wirkstoffs - im Gegensatz zu derjenigen der erfindungsgemässen Pellets - nicht sicher erreicht werden kann.

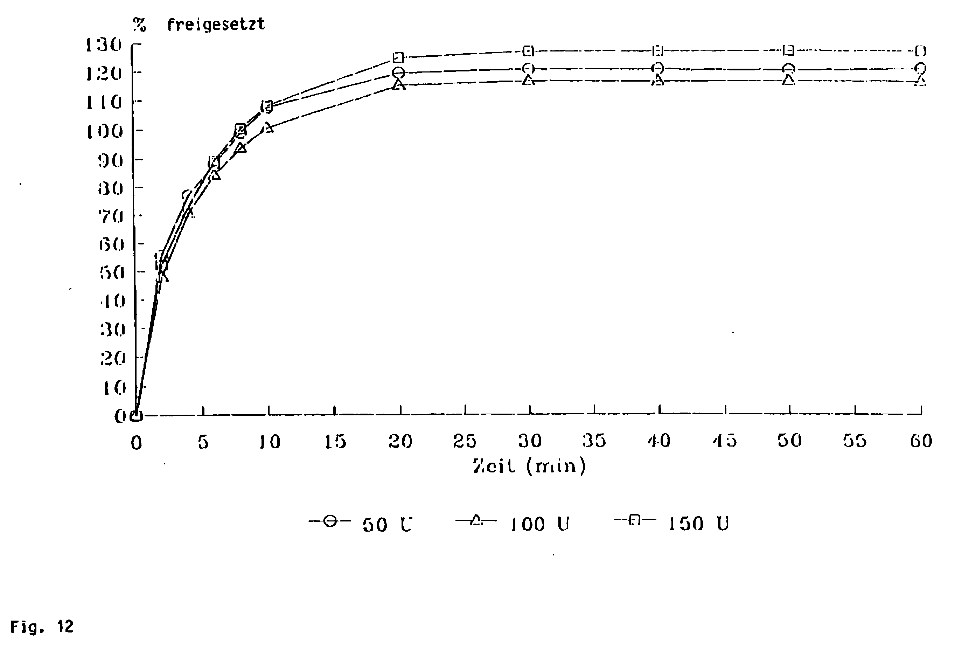

In den Fig. 12-14 wird das Freigabeverhalten von Minocyclin-HCl analog der Fig. 9-11 gemessen mit dem Unterschied, dass Minocinkapseln mit 50 mg Minocyclin-HCl untersucht wurden. Es zeigt sich dabei, dass sich das Freigabeverhalten im Verhältnis zu demjenigen der 100 mg enthaltenden Minocinkapseln nicht wesentlich verändert.

## Patentansprüche

1. pH-unabhängige, den Wirkstoff schnell freigebende, ungecoatete Pellets, enthaltend mindestens 90 Gew.-% bis zu 99,5 Gew.-% Minocyclin-HCl oder ein anderes pharmazeutisch verträgliches Minocyclinsalz und 0,5-10 Gew.-% Polyethylenglykol als Pelletierhilfsmittel.

2. Pellets nach Patentanspruch 1, enthaltend eine Politur aus 2,9-6 Gew.-% Polyethylenglykol.

3. Pellets nach Patentanspruch 1 oder 2, enthaltend als Pelletierhilfsmittel Polyethylenglykol 2000 bis 20000, insbesondere Polyethylenglykol 2000, 3000, 4000, 6000, 10000 oder 20000, eine Kombination oder ein Gemisch davon.

4. Pellets nach einem der Patentansprüche 1-3, enthaltend mindestens 93 Gew.-% Minocyclin-Hydrochlorid und höchstens 7 Gew.-% Polyethylenglykol.

5. Pellets nach einem der Patentansprüche 1-4, enthaltend 6,8 Gew.-% Polyethylenglykol 4000 oder 3,9 Gew.-% Polyethylenglykol 4000 und 2,9 Gew.-% Polyethylenglykol 10000.

6. Pellets nach einem der Patentansprüche 1-4, enthaltend mindestens 96 Gew.-% Minocyclin-Hydrochlorid und höchstens 4 Gew.-% Polyethylenglykol, vorzugsweise Polyethylenglykol 4000.

7. Pellets nach einem der Patentansprüche 1-6, gekennzeichnet durch eine praktisch vollständige in vitro-Freigabe des Wirkstoffs innert weniger als 10 Minuten, insbesondere weniger als 5 Minuten, sowohl bei pH 1,2 als auch bei pH 7,4.

8. Pellets nach einem der Patentansprüche 1-7, gekennzeichnet durch einen Durchmesser von 0,5-1,4 mm, vorzugsweise 0,8-1,2 mm.

9. Pellets nach einem der Patentansprüche 1-8 mit reproduzierbaren pharmakokinetischen Eigenschaften mit geringer Streuung der erzielbaren Plasmaspiegel, vorzugsweise mit einem Variationskoeffizienten von <20 %, besonders bevorzugt von <14 %, der erzielbaren Plasmaspiegel.

10. Oral verabreichbares Arzneimittel, enthaltend in einer Kapsel, z.B. Gelatinekapsel, mindestens 50 mg, vorzugsweise 50 oder 100 mg Minocyclin-HCl in Pellets nach einem der Patentansprüche 1-9.

11. Verfahren zur Herstellung von Pellets nach einem der Patentansprüche 1-9, dadurch gekennzeichnet, dass man Wasser oder ein Wasser/Alkoholgemisch und Polyethylenglykol in einem Hochgeschwindigkeitsmischer mit Minocyclin-HCl oder einem anderen pharmazeutisch unbedenklichen Minocyclinsalz solange mischt, bis Pellets der gewünschten Grösse entstanden sind, die Pellets lufttrocknet und nach der Grösse über Siebe klassiert und sie gegebenenfalls ein weiteres Mal mit im Wasser oder Wasser/Alkoholgemisch gelöstem Polyethylenglykol rundet, trocknet und siebt.

## Claims

1. pH-independent uncoated pellets, setting free quickly the active substance, containing at least 90% by weight to 99,5% by weight minocycline-HCl or another pharmaceutically acceptable salt of minocycline and 0,5-10% by weight of polyethylenglycol as a pelletising agent.

2. Pellets according to claim 1, having a polish of 2,9-6% by weight of polyethylenglycol.

3. Pellets according to claim 1 or 2, containing as pelletising agent polyethylenglycol 2000 to 20000, especially polyethylenglycol 2000, 3000, 4000, 6000, 10000 or 20000, a combination or a mixture thereof.

4. Pellets according to any one of the claims 1-3, containing at least 93% by weight minocycline-hydrochlorid and highest 7% by weight polyethylenglycol.

5. Pellets according to any one of the claims 1-4, containing 6,8% by weight polyethylenglycol 4000 or 3,9% by weight polyethylenglycol 4000 and 2,9% by weight polyethylenglycol 10000.

6. Pellets according to any one of the claims 1-4, containing at least 96% by weight minocycline-hydrochlorid and highest 4% by weight polyethylenglycol, preferably polyethylenglycol 4000.

7. Pellets according to any one of the claims 1-6, characterised in that the active substance is set free in vitro practically completely within less than 10 minutes, especially less than 5 minutes, at pH 1,2 as well as at pH 7,4.

8. Pellets according to any one of the claims 1-7, characterised by having a diameter of 0,5-1,4 mm, preferably 0,8-1,2 mm.

9. Pellets according to any one of the claims 1-8, with reproducible pharmaco-kinetic properties with a small variation of the achievable plasma levels, preferably with a variation-coefficient of <20%, especially preferred of <14 %, of the achievable plasma levels.

10. Oral administrable medicament, containing in a capsule, for example a gelatine capsule, at least 50 mg, preferably 50 or 100 mg minocycline-HCl as pellets according to any one of the claims 1-9.

11. Process for the production of pellets according to any one of the claims 1-9, characterised by mixing water or a water/alcohol mixture and polyethylenglycol in a high speed mixer together with minocycline-HCl or another pharmaceutically acceptable salt of minocycline for a time long enough to form pellets of the desired size, air-drying the pellets and classifying the pellets with sieves according to their size and optionally rounding the pellets a further time with polyethylenglycol dissolved in water or in a water/alcohol mixture, drying and sieving.

## Revendications

1. Des pellets non-enrobées, ph-indépendantes, libérant rapidement leur substance active, contenant au moins 90 % en poids jusqu'à 99,5 % en poids de minocycline-HCl ou un autre sel de minocycline pharmaceutiquement acceptable et 0,5-10 % en poids de polyéthylène-glycol comme adjuvant pour la formation des pellets.

2. Des pellets selon la revendication 1, contenant un vernis de 2,9 - 6 % en poids de polyéthylène-glycol.

3. Des pellets selon la revendication 1 ou 2, contenant en tant qu'adjuvant pour la formation des pellets du polyéthylène'glycol 2000 à 20000, en particulier du polyéthylène-glycol 2000, 3000, 4000, 6000, 10000 ou 20000, leur combinaison ou leur mélange.

4. Des pellets selon l'une quelconque des revendications 1 à 3, contenant au moins 93 % en poids de minocycline-hydrochlorure et au maximum 7 % en poids de polyéthylène-glycol.

5. Des pellets selon l'une quelconque des revendications 1 à 4, contenant 6,8 % en poids de polyéthylèneglycol 4000 ou 3,9 % en poids de polyéthylène-glycol 4000 et 2,9 % en poids de polyéthylène-glycol 10000.

6. Des pellets selon l'une quelconque des revendications 1 à 4, contenant au moins 96 % en poids de minocycline-hydrochlorure et au maximum 4 % en poids de polyéthylène-glycol, de préférence du polyéthylène-glycol 4000.

7. Des pellets selon l'une quelconque des revendications 1 à 6, caractérisées en ce que la substance active est pratiquement complètement libérée in vitro en moins de 10 minutes, en particulier moins de 5 minutes, à une valeur pH de 1,2 ainsi qu'à une valeur pH de 7,4.

8. Des pellets selon l'une quelconque des revendications 1 à 7, caractérisées par un diamètre de 0,5-1,4 mm, de préférence de 0,8-1,2 mm.

9. Des pellets selon l'une quelconque des revendications 1 à 8 avec des propriétés pharmacocinétiques reproductibles avec une faible variation des niveaux de plasma qu'on peut obtenir, de préférence avec un coefficient de variation de <20%, particulièrement préféré de <14% des niveaux de plasma obtenables.

10. Un médicament administrable par voie orale, contenant dans une gélule, par exemple une gélule de gélatine, au moins 50 mg, de préférence 50 ou 100 mg de minocycline-HCl telles que les pellets selon l'une quelconque de revendications 1 à 9.

11. Un procédé de fabrication de pellets selon l'une quelconque des revendications 1 à 9, caractérisé en ce qu'on mélange de l'eau ou un mélange eau/alcool et du polyéthylène-glycol dans un mélangeur à haute vitesse avec du minocycline-HCl ou un autre sel de minocycline pharmaceutiquement acceptable pendant un temps assez long jusqu'à formation de pellets de la taille souhaitée, séchage des pellets à l'air, et classification des pellets sur des tamis selon leur taille et, optionnellement encore une fois arrondissement avec du polyéthylène-glycol dissout dans de l'eau ou dans un mélange d'eau/alcool, séchage et tamisage.
